# EUROPÄISCHE PATENTANMELDUNG

(11) **EP 2 653 178 A1**
(43) Veröffentlichungstag der Anmeldung: **23.10.2013**
(21) Anmeldenummer: 12164284.7
(22) Anmeldetag: 16.04.2012
(51) Int. Cl.: A61M 1/16, A61M 1/36

(54) **Sicherheitseinrichtung für eine extrakorporale Blutbehandlung**

(71) Anmelder: Zentrum für biomedizinische Technologie der Donau- Universität Krems, 3500 Krems (AT)
(72) Erfinder: Falkenhagen, Dieter, 3500 Krems (AT); Brandl, Martin, 3620 Spitz (AT); Hartmann, Jens, 3511 Furth (AT)
(74) Vertreter: Patentanwaltskanzlei Matschnig & Forsthuber OG

(57) **Zusammenfassung**

Blutreinigungsvorrichtung (100,200) aufweisend einen extrakorporalen Blutkreislauf (102,202) mit einem Blutzulauf (103,203) zu einer Dialyseeinheit (105,205) für von einem Patienten entnommenes Blut und einem Blutablauf (104,204) von der Dialyseeinheit (105,205) für in den Patienten zurückzuführendes Blut, wobei die Dialyseeinheit (105,205) einen Zulauf (107,207) für eine Dialysierlösung und einen Dialysatablauf (108,208) umfasst, gekennzeichnet durch einen Regelkreis zur Regelung des Säurebasengleichgewichts des Blutes, wobei dem Blut mittels einer wässrigen Bicarbonat-hältigen Lösung Bicarbonat zugeführt ist, wobei dem Regelkreis zumindest ein Messmittel (124,124a,224,224a) zum Messen eines Blutpufferparameters zugeordnet ist, wobei der Blutpufferparameter ausgewählt ist aus der Gruppe bestehend aus Bicarbonat-Konzentration und CO₂-Partialdruck, und wobei ein dem gemessenen Istwert des Blutpufferparameters entsprechendes Signal einer Regeleinrichtung (125,225) zuführbar ist, welche den Istwert des Blutpufferparameters über die Zufuhr des Bicarbonats auf einen vorgebbaren Sollwert oder Sollwertbereich regelt, und zumindest ein pH-Messmittel (150a,250a), welches im extrakorporalen Blutkreislauf (102,202) stromauf der Dialyseeinheit (105,205) angeordnet ist, wobei ein dem pH-Wert entsprechendes Signal einer Alarmeinrichtung (130,230) zuführbar ist, welche bei Überschreiten oder Unterschreiten eines vorgebbaren pH-Werts oder pH-Wert-Bereichs ein Alarmsignal auslöst.

## Beschreibung

Die Erfindung betrifft eine Blutreinigungsvorrichtung, aufweisend einen extrakorporalen Blutkreislauf mit einem Blutzulauf zu einer Dialyseeinheit für von einem Patienten entnommenes Blut und einem Blutablauf von der Dialyseeinheit für in den Patienten zurückzuführendes Blut, wobei die Dialyseeinheit einen Zulauf für eine Dialysierlösung und einen Dialysatablauf umfasst.

Der Organismus eines Säugetiers funktioniert nur dann einwandfrei, wenn möglichst immer die gleichen physikalischen und chemischen Bedingungen vorherrschen. Besonders wichtig ist die Aufrechterhaltung des Säure-Basen-Haushalts, der in einem sehr engen physiologischen Bereich durch körpereigene Mechanismen konstant gehalten wird. Das Kohlensäure-Bicarbonat-Puffersystem bildet den Großteil der Gesamtpufferkapazität des Blutes und spielt beim Ausgleichen von pH-Schwankungen des Blutes eine wesentliche Rolle. Saure Stoffwechselprodukte, die sich ständig im Körper bilden, werden üblicherweise neutralisiert, indem sie entweder über die Nieren ausgeschieden werden bzw. indem über die Lungen CO₂ abgeatmet wird. Beim Menschen liegt der pH-Wert des Blutes in einem sehr engen Bereich zwischen 7,35 - 7,45. Liegt der pH-Wert unter 7,35 spricht man von einer Azidose, liegt er über 7,45, dann spricht man von einer Alkalose. Sowohl Azidose als auch Alkalose sind gefährliche Störungen des Säure-Basen-Haushalts, die neben erheblichen körperlichen Beschwerden (z.B. Kopfschmerzen, Muskelkrämpfe, Knochenschädigung, Übelkeit und Müdikeit) auch zu akut lebensgefährlichen Zuständen führen können.

Bei Patienten mit chronischer Niereninsuffizienz und Dialysepatienten können diese sauren Stoffwechselprodukte nicht mehr ausreichend über die Niere ausgeschieden werden. Die fehlende Nierenfunktion kann nur begrenzt von den Lungen ausgeglichen werden. Die Übersäurung des Blutes durch nicht abgeführte saure Stoffwechselprodukte führt bei Dialysepatienten daher zur metabolischen Azidose, welche durch ein Absinken des pH-Werts des Blutes und der Bicarbonatkonzentration im Blut gekennzeichnet ist. Während einer extrakorporalen Blutreinigung wird das Blut eines Patienten daher nicht nur von Schadstoffen gereinigt, sondern es wird auch die entstandene Übersäuerung des Blutes ausgeglichen.

Früher wurde zur Azidosekorrektur Acetat verwendet, welches im menschlichen Körper zum basischen Puffer Bicarbonat umgesetzt wird. Da es aufgrund dieser Umwandlung des Acetats im Körper zu einer verzögerten Wirkung kommt, wird heutzutage einer Azidose standardmäßig mit Bicarbonat (Bicarbonation, HCO₃⁻) entgegengewirkt, welches entweder an der Membrangrenzfläche des Dialysefilters mittels einer Bicarbonat-hältigen Dialysierflüssigkeit (bei Hämodialyse und Hämodiafiltration) oder mittels einer Bicarbonat-hältigen Infusionslösung (bei Hämodiafiltration und Hämofiltration) dem Blut nach der Filterpassage zugeführt wird. Das Bicarbonat reagiert mit den (sauren) H⁺ - Ionen im Blut zu Kohlensäure, welche ihrerseits zu H₂O (Wasser) und CO₂ (Kohlendioxid) dissoziiert:

CO₂ + H₂O <==> H₂CO₃ <==> H⁺ + HCO₃-

Das Kohlendioxid wird über die Lunge abgeatmet und das Wasser über die Dialyse bzw. über Filtration entfernt. Dialysen werden heutzutage hauptsächlich als Bicarbonatdialysen durchgeführt. Häufig wird auch eine Mischung aus Bicarbonat und Acetat verwendet, wobei die Dialysierflüssigkeit unmittelbar vor der Passage durch den Dialysator (Dialysefilter) aus einer konzentrierten Acetatlösung (saure Komponenten) und einer konzentrierten Bicarbonatlösung (basische Komponente) und Wasser zusammengemischt wird.

Zu Beginn einer extrakorporalen Blutreinigung, welche auf dem Prinzip einer Hämodialyse, Hämofiltration oder Hämodiafiltration beruhen kann, wird üblicherweise der Säuregrad im Blut mittels Blutgasanalyse bestimmt und anhand des erhaltenen Wertes die Bicarbonatzufuhr eingestellt. Eine zu starke Korrektur des Säure-Basen-Gleichgewichts in Richtung basischen Bereich kann wiederum zu einer Gefährdung des Patienten führen. Eine Bicarbonat-Dialyse wird nahezu ausschließlich bei der Akutdialyse und bei der chronischen Hämodialyse angewandt.

Entgleisungen des Säurebasengleichgewichts und Schwankungen des Blut-pH-Werts stellen für einen Dialysepatienten ein hohes Sicherheits- und Gesundheitsrisiko dar und können zu lebensbedrohlichen Zuständen führen. Es ist daher wünschenswert, die Konstanz des pH-Werts des Blutes wiederherzustellen bzw. das Säurebasengleichgewicht aufrechtzuerhalten.

Es ist daher eine Aufgabe der Erfindung, Maßnahmen bereitzustellen, die einer Störung des Säurebasengleichgewichts entgegenwirken und eine Konstanthaltung des Blut-pH-Werts bewirken, um einerseits das Wohlbefinden des Patienten wiederherzustellen und zu bewahren und andererseits das Auftreten gefährlicher physiologischer Zustände zu verhindern. Insbesondere ist es eine Aufgabe der Erfindung dem Auftreten einer Azidose bzw. einer Alkalose, deren Auswirkungen nicht nur eine hohe Belastung für den Organismus darstellen sondern auch lebensbedrohlich sein können, vorzubeugen.

Die Aufgabe wird durch eine Blutreinigungsvorrichtung wie eingangs genannt gelöst, welche erfindungsgemäß durch einen Regelkreis zur Regelung des Säurebasengleichgewichts des Blutes gekennzeichnet ist, wobei dem Blut mittels einer wässrigen Bicarbonat-hältigen Lösung Bicarbonat zugeführt ist, wobei dem Regelkreis zumindest ein Messmittel zum Messen eines Blutpufferparameters zugeordnet ist, wobei der Blutpufferparameter ausgewählt ist aus der Gruppe bestehend aus Bicarbonat-Konzentration und CO₂-Partialdruck, und wobei ein dem gemessenen Istwert des Blutpufferparameters entsprechendes Signal einer Regeleinrichtung zuführbar ist, welche den Istwert des Blutpufferparameters über die Zufuhr des Bicarbonats auf einen vorgebbaren Sollwert oder Sollwertbereich regelt, und wobei die Blutreinigungsvorrichtung ferner durch zumindest ein pH-Messmittel gekennzeichnet ist, welches im extrakorporalen Blutkreislauf stromauf der Dialyseeinheit angeordnet ist, wobei ein dem pH-Wert entsprechendes Signal einer Alarmeinrichtung zuführbar ist, welche bei Überschreiten oder Unterschreiten eines vorgebbaren pH-Werts oder pH-Wert-Bereichs ein Alarmsignal auslöst.

Dank der Erfindung wird bei einer extrakorporalen Blutreinigung eine Regelung bereitgestellt, mittels welcher Störungen des Säurebasengleichgewichts, wie eine Azidose oder eine Alkalose, vorgebeugt wird und die Konstanthaltung des physiologischen pH-Wert des Blutes und somit des Säurebasengleichgewichts gewährleistet ist. Dank der Erfindung ist es ferner möglich, Störungen des Säurebasengleichgewichts sofort zu erkennen und diesen entgegenzuwirken, so dass das Risiko schwerwiegender oder sogar lebensbedrohlicher Zustände so gering als möglich gehalten werden kann. Die Erfindung trägt somit zu einer wesentlichen Verbesserung der Patientensicherheit bei.

Die erfindungsgemäße spezifische Kombination eines Regelkreises mit einem Messmittel zum Messen eines Blutpufferparameters und eines stromauf der Dialyseeinheit angeordneten pH-Messmittels ermöglicht das Unterscheiden zwischen einer kompensierten und einer nicht kompensierten Alkalose bzw. Azidose. Eine nicht-kompensierte Alkalose bzw. Azidose tritt immer dann auf, wenn es zur Überschreitung des physiologischen pH-Werts kommt, das heißt, wenn ein pH von 7,45 überschritten bzw. ein pH von 7,35 unterschritten wird. Bei einer nicht-kompensierten Alkalose bzw. Azidose droht große Gefahr für den Patienten. Sinngemäß weicht bei einer kompensierten Alkalose bzw. einer kompensierten Azidose der Blutpufferparameter (d.h. die Bicarbonat-Konzentration bzw. der CO₂-Partialdruck) vom Normbereich ab, während sich der pH-Wert im Normbereich befindet. Bei einer nicht-kompensierten Alkalose bzw. einer nicht-kompensierten Azidose befinden sich sowohl der Blutpufferparameter (d.h. die Bicarbonat-Konzentration bzw. der CO₂-Partialdruck) als auch der pH-Wert außerhalb der entsprechenden Normbereiche. Ein stromauf der Dialyseeinheit gemessener pH-Wert entspricht dem aktuell intrakorporal vorliegenden pH-Wert des Patientenblutes und ermöglicht ein schnelles Erkennen einer nicht-kompensierten Alkalose bzw. Azidose.

Die EP0097366 beschreibt eine Dialysevorrichtung mit geregelter Elektrolytzusammensetzung der Dialysierlösung, wobei Detektoren zur Messung der im Blut vorliegenden Elektrolyte, der Leitfähigkeit oder des pH-Werts vorgesehen sind. Die Merkmale der vorliegenden Erfindung samt den damit einhergehenden Vorteilen hinsichtlich der Patientensicherheit sind darin weder geoffenbart noch in irgendeiner Form angedacht.

Die HCO₃⁻ -Konzentration (Bicarbonat-Konzentration) und der CO₂-Partialdruck (pCO₂) sind Blutpufferparameter, die in der klinischen Anwendung in gängiger Art und Weise zur Beurteilung des Säure-Basen-Haushalts eines Säugers, insbesondere Menschen, herangezogen werden. Für den Ausdruck Säurebasengleichgewicht wird synonym der Ausdruck Säure-Basen-Haushalt verwendet. Anhand der beiden genannten Blutpufferparameter können der Ursprung und die Ausprägung einer Alkalose bzw. einer Azidose festgestellt werden. Metabolische Störungen können charakteristischerweise anhand der Abweichungen in der HCO₃⁻ -Konzentration im Blut nachgewiesen und im Schweregrad beurteilt werden. Der pH-Wert, die HCO₃⁻ -Konzentration und der CO₂-Partialdruck stehen über die Henderson-Hasselbach-Gleichung miteinander in Verbindung.

Aufgrund der im Körper stattfindenden Kompensationsmechanismen schließt ein fast normaler pH-Wert das Vorhandensein eines Säure-Basen-Ungleichgewichts nicht aus. Für die Beurteilung des Säure-Basen-Gleichgewichts ist das Messmittel zum Messen eines Blutpufferparameters zur Messung der HCO₃⁻ -Konzentration oder des CO₂-Partialdrucks vorgesehen ist. In der vorliegenden Erfindung wird die aktuelle HCO₃⁻ -Konzentration bzw. der CO₂-Partialdruck im Blut laufend gemessen und der Istwert durch die Zufuhr der wässrigen Bicarbonat-Lösung auf einen vorgebbaren Sollwert oder Sollbereich geregelt.

Die Normalwerte der HCO₃⁻ -Konzentration liegen typischerweise bei 21 - 26 mmol/l und diejenigen des CO₂-Partialdrucks bei 35 - 45 mm Hg. Der vorgebbare Sollwert oder Sollbereich entspricht typischerweise folglich diesen Normalwerten. Je nach Zustand des Patienten kann der Sollbereich auch enger als der zuvor genannte jeweilige typische Normalbereich vorgegeben werden.

Um einen Bediener auf den Zustand einer nicht-kompensierten Alkalose bzw. einer nicht-kompensierten Azidose aufmerksam zu machen ist es erfindungsgemäß vorgesehen, dass bei einem Überschreiten oder Unterschreiten eines vorgebbaren pH-Werts oder pH-Wertbereichs durch die Alarmeinrichtung ein Alarmsignal ausgelöst wird. Das Alarmsignal kann beispielsweise ein akustisches oder optisches Signal sein. Aufgrund des Signals kann ein Bediener sofort entsprechende Gegenmaßnahmen einleiten. Darüber hinaus kann das Auslösen eines Alarms ein Anhalten des Blutreinigungsgeräts und somit des Blutreinigungsvorgangs bewirken.

Alarmeinrichtungen und Regeleinrichtungen wie sie bei dieser Erfindung eingesetzt werden, sind einem Fachmann auf dem Gebiet extrakorporaler Blutreinigungsgeräte hinlänglich bekannt. Die Alarmeinrichtung und die Regeleinrichtung sind typischerweise der zentralen Geräteelektronik und - steuerung zugeordnet, können aber auch völlig eigenständige elektronische Bauteile sein.

pH-Messmittel zum Messen des Blut-pH-Werts während einer extrakorporalen Blutreinigung sind dem einschlägigen Fachmann bekannt. Ein für die Anwendung in der vorliegenden Erfindung geeignetes pH-Messmittels ist beispielsweise ein pH-Sensor hergestellt von der Firma Lazar Research Laboratories, Inc., z.B. ein pH-Sensor vom Typ FTPH-2SXS.

Geeignete Messmittel zum Messen eines Blutpufferparameters, die in dieser Erfindung zur Anwendung kommen können, sind ebenfalls im Stand der Technik beschrieben. Beispielsweise sind in der EP 0759 551 B1, CA 2173672 A2 und der US 2009/027095 A1 Sensoren zum Messen der HCO₃⁻ -Konzentration und/oder des CO₂-Partialdrucks geoffenbart, die für die Ausführung dieser Erfindung geeignet sind. Diese Sensoren eignen sich auch zur laufenden Messung der Blutpufferparameter des im extrakorporalen Blutkreislauf zirkulierenden Bluts. Bei bestimmten Ausführungen kann das Messmittel in den extrakorporalen Blutkreislauf eingesetzt sein und das Messmittel steht in Kontakt mit dem vorbeiströmenden Blut. In diesen Fällen ergeben sich hohe Ansprüche an die Beschaffenheit des Messmittels hinsichtlich Sterilität und Biokompatibilität. Alternativ hierzu kann bei anderen Ausführungen eine geringe Menge Blut über eine Bypass-Leitung vom extrakorporalen Blutkreislauf zu einem Messmittel geleitet und anschließend verworfen werden.

Unter "zumindest ein Messmittel" versteht sich im Rahmen dieser Offenbarung, dass an einer bestimmten Position nur ein Messmittel zum Messen des pH-Werts bzw. Blutpufferparameters oder auch mehr als ein Messmittel, z.B. zwei oder mehr wesensgleiche Messmittel, welche alternierend oder zeitgleich messen, angeordnet sind.

Unter dem Begriff "laufende Messung" ist erfindungsgemäß eine Messung des Blutpufferparameters bzw. des pH-Werts in regelmäßigen Zeitintervallen zu verstehen, wobei die Zeitintervalle vorteilhafterweise ausreichend kurz gehalten werden, um den Säurebasenhaushalt ausreichend engmaschig zu überwachen. Ausreichend kurze Zeitabstände sind solche, bei denen gewährleistet werden kann, dass eine Entwicklung in Richtung eines unphysiologischen Zustands des Patienten rechtzeitig erkannt und dieser entgegengewirkt werden kann. Bei den meisten Anwendungen ist ein Zeitintervall von 30 bis 60 Minuten ausreichend. Bei gewissen Patientengruppen, z.B. Patienten mit Lebererkrankungen, welche das bei einer Citrat-Antikoagulation zugeführte Citrat nur beschränkt in Bicarbonat umwandeln können (siehe weiter unten), ist der Zeitabstand zwischen zwei aufeinanderfolgenden Messungen kürzer gewählt, z.B. 15 Minuten.

Der Begriff "extrakorporale Blutreinigung" umfasst verschiedene Blutreinigungsverfahren auf Membranbasis wie die Hämodialyse, die (sogenannte online-) Hämofiltration und die (sogenannte online-) Hämodiafiltration sowie eine Kombination dieser Verfahren mit Verfahren basierend auf Adsorption wie Hämoperfusion, Plasmasorption, etc, wobei all diese Verfahren dem einschlägigen Fachmann bekannt sind.

Die Regelung des Säurebasengleichgewichts erfolgt über die Zufuhr von Bicarbonat (HCO₃⁻) mittels einer Bicarbonat-hältigen wässrigen Lösung, da dieses, wie oben erwähnt, auch eine wesentliche basische Komponente des körpereigenen Blutpuffersystems und ein in der klinischen Anwendung lange erprobtes und zugelassenes Agens darstellt.

Bei einer ersten vorteilhaften Ausführungsform handelt es sich bei der wässrigen Bicarbonat-Lösung um eine Dialysierlösung, die über den Zulauf für Dialysierlösung in die Dialyseeinheit (auch als Dialysator oder Dialysefilter bezeichnet) geleitet wird. Diese Ausführungsform ist insbesondere für die Hämodialyse und die Hämodiafiltration zweckmäßig. Bicarbonat wird dem Blut über die Dialysierflüssigkeit zugeführt (Bicarbonat-Dialyse). Die Bicarbonat-Dialyse an sich ist dem einschlägigen Fachmann bekannt. Zweckmäßigerweise wird durch den erfindungsgemäßen Regelkreis die Zusammensetzung der Dialysierlösung geändert. Bei einer gängigen und dem einschlägigen Fachmann bekannten Ausführung wird unmittelbar vor der Passage durch den Dialysator die Dialysierlösung aus einer konzentrierten sauren Komponente (z.B. Acetatlösung) und einer konzentrierten basischen Komponente (Bicarbonat-Lösung) und den restlichen Komponenten der Dialysierlösung zusammengemischt. Durch Erhöhen oder Drosseln der beigemischten Menge an Bicarbonat lässt sich die Konzentration des Bicarbonats in der Dialysierlösung entsprechend variieren.

Bei einer weiteren vorteilhaften Ausführungsform handelt es sich bei der wässrigen Bicarbonat-Lösung, mittels welcher dem Blut Bicarbonat zugeführt ist, um eine Infusionslösung. Diese Ausführungsform ist insbesondere für die Hämofiltration, die Hämodiafiltration und die Online-Hämodiafiltration zweckmäßig. Das Bicarbonat kann hier mittels einer Substitutionslösung (auch als Substitutionsmedium oder Substituat bezeichnet) vorzugsweise durch Infusion in den extrakorporalen Blutkreislauf dem Blut zugeführt werden.

Das Bicarbonat wird über ein Mischsystem dem Dialysat bzw. dem Substituat kurz vor dem Filter vorzugsweise in einem Verhältnis von 1:28 bis 1:32 zugesetzt. Derartige Mischsysteme sind dem Fachmann bekannt. Da das Bicarbonat erst kurz vor der Filterpassage zugemischt wird, wird ein Ausfallen unlöslicher Carbonat-Verbindungen verhindert.

Bei den meisten Patienten, die einer extrakorporalen Blutreinigung bedürfen, ist eine Hemmung der Blutgerinnung erforderlich. Als derzeitiger Standard wird in der klinischen Behandlung Heparin eingesetzt. Eine Alternative zur Heparinmethode stellt die Antikoagulation mit Citrat dar. Bei einer Ausführungsform weist die Blutreinigungsvorrichtung daher eine Citrat-Antikoagulationseinsrichtung auf, wobei dem extrakorporalen Blutkreislauf an einer Citratzufuhrstelle stromauf der Dialyseeinheit Citrat und an einer Substitutionsmedium zufuhrstelle stromab der Dialyseeinheit Substitutionsmedium zugeführt ist.

Bei einer Antikoagulation mit Citrat können Störungen im Säure-Basen-Haushalt besonders leicht auftreten. Bei dieser Form der Antikoagulation wird dem Blut ständig Citrat zugeführt, welches im Körper zu Bicarbonat umgesetzt wird und zu unphysiologischen Blut-pH-Werten führen kann, die eine echte Gefahr für den Patienten darstellen. Durch eine Anhäufung von Bicarbonat im Organismus des Patienten kann eine Alkalose in einem lebensgefährlichen Ausmaß entstehen. Auch eine Azidose ist möglich, die insbesondere dann auftritt, wenn eine nicht funktionierende Leber, z.B. bei Patienten mit Leberinsuffizienz oder Leberversagen, nicht in der Lage ist, das Citrat in ausreichendem Maße in Bicarbonat umzuwandeln, wodurch das Citrat im Blut verbleibt. Die Erfindung ist daher besonders vorteilhaft, wenn das Blut mit Citrat antikoaguliert wird und ermöglicht es, die Konstanz des pH-Werts des Blutes wiederherzustellen bzw. das Säurebasengleichgewicht aufrechtzuerhalten.

Dialyseverfahren mit Citrat-Antikoagulation sind aus dem Stand der Technik bekannt, z.B. aus WO 91/06326, US 2007/066928 A1, US 2004/133145A1, DE 101 14 283 C2 und WO 2009/026603. Bei einer Blutreinigungsvorrichtung gemäß der Erfindung, die mit einer Citrat-Antikoagulationseinrichtung ausgestattet ist, kann die Bicarbonat-hältige Lösung entweder die Dialysierlösung oder das Substitutionsmedium sein.

Ist der Blutreinigungsvorrichtung eine Citrat-Antikoagulationseinrichtung zugeordnet, dann ist das zumindest eine pH-Messmittel vorzugsweise im extrakorporalen Blutkreislauf stromauf der Citratzufuhrstelle angeordnet. Der an dieser Position gemessene pH-Wert entspricht dem aktuell intrakorporal vorliegenden pH-Wert des Patientenblutes und ermöglicht ein schnelles Gegensteuern im Falle eines unphysiologischen pH-Werts.

Bei einer vorteilhaften Ausführungsform der Erfindung ist das zumindest eine Messmittel zum Messen eines Blutpufferparameters im extrakorporalen Blutkreislauf stromauf der Dialyseeinheit angeordnet. Der an dieser Position gemessene Wert des Blutpufferparameters spiegelt das aktuell intrakorporal vorliegende Säurebasengleichgewicht wider und ermöglicht ein rasches Gegensteuern im Falle einer Störung des Säurebasengleichgewichts.

Ist der Blutreinigungsvorrichtung eine Citrat-Antikoagulationseinrichtung zugeordnet, dann ist es zweckmäßig, wenn das zumindest eine Messmittel zum Messen eines Blutpufferparameters im extrakorporalen Blutkreislauf stromauf der Citratzufuhrstelle angeordnet ist. Der Vorteil dieser Anordnung liegt darin, dass eine stromauf der Citratzufuhrstelle im extrakorporalen Blutkreislauf durchgeführte Messung des Blutpufferparameters das aktuell intrakorporal vorliegende Säure-Basen-Gleichgewicht repräsentiert, welches nach Zugabe von Citrat verändert wird. Eine unzureichende Umwandlung des Citrats in Bicarbonat in der Leber des Patienten und die damit einhergehende Gefahr einer Alkalose kann sofort erkannt und mittels des erfindungsgemäßen Regelmechanismus durch Drosseln der Bicarbonat-Zufuhr umgangen werden. Bicarbonat (HCO₃⁻)wird bei einer Untervariante dieser Ausführungsform über das Substitutionsmedium zugeführt. Da das Substitutionsmedium bei der Citrat-Antikoagulation üblicherweise Calcium-Ionen enthält, welche fallweise mit dem Bicarbonat als Calciumcarbonat ausfallen können, kann das Bicarbonat alternativ auch in Form einer wässrigen Substitutionslösung über einen weiteren, separaten Zugang in den extrakorporalen Blutkreislauf infundiert wird.

Alternativ zur oben genannten Ausführungsform, bei welcher das Messmittel zum Messen eines Blutpufferparameters stromauf der Dialyseeinheit bzw. stromauf der Citratzufuhrstelle angeordnet ist, ist bei einer weiteren vorteilhaften Ausführungsform das zumindest eine Messmittel zum Messen eines Blutpufferparameters im Dialysatablauf angeordnet. Die Anordnung im Dialysatablauf hat den Vorteil, dass das Messmittel nicht direkt mit dem Blut in Kontakt kommt und daher im Gegensatz zu einem blutseitig angeordneten Messmittel keine wie immer geartete Kontamination des Patienten, z.B. durch Keime oder nichtbiokompatible Messmittelmaterialien, entstehen kann. Die klinische Zulassung des Messmittels vereinfacht sich dadurch deutlich. Weiters ist ein Anhaften von Zellen und Proteinen auf der Messmitteloberfläche im Dialysat ausgeschlossen, wodurch das Messmittel eine gute Langzeitstabilität aufweist.

Als Weiterbildung der oben genannten Ausführungsformen, in welchen zumindest ein erstes Blutpuffermessmittel in einer ersten Position angeordnet ist, kann als weiterführende Sicherheitsmaßnahme dem Regelkreis an einer zweiten Position zumindest ein zusätzliches zweites Messmittel zum Messen eines Blutpufferparameters zugeordnet sein. Das zweite zusätzliche Messmittel dient entweder zum Erkennen von Fehlfunktionen des zur Regelung fungierenden ersten Messmittels oder das zweite zusätzliche Messmittel kann wie das erste Messmittel ebenfalls in die Regelung einbezogen sein.

Bei einer ersten Variante ist bei Vorhandensein zumindest eines ersten Blutpufferparametermessmittels stromauf des Dialysefilters bzw. stromauf der Citratzufuhrstelle dem Regelkreis zumindest ein zweites zusätzliches, im Dialysatablauf angeordnetes Messmittel zum Messen eines Blutpufferparameters zugeordnet. Bei einer zweiten Variante ist bei Vorhandensein zumindest eines ersten Blutpufferparametermessmittels im Dialysatablauf dem Regelkreis zumindest ein zweites zusätzliches Messmittel zum Messen eines Blutpufferparameters zugeordnet, welches im extrakorporalen Blutkreislauf stromauf der Dialyseeinheit angeordnet ist. Ist der Blutreinigungsvorrichtung eine Citrat-Antikoagulationseinrichtung zugeordnet, dann ist es zweckmäßig, wenn das zusätzliche Messmittel im extrakorporalen Blutkreislauf stromauf der Citratzufuhrstelle angeordnet ist.

Darüber hinaus kann dem Regelkreis in einer Weiterbildung der zuvor genannten Ausführungsformen zumindest ein zusätzliches Messmittel zum Messen eines Blutpufferparameters zugeordnet sein, welches im extrakorporalen Blutkreislauf stromab der Dialyseeinheit angeordnet ist. Ist der Blutreinigungsvorrichtung eine Citrat-Antikoagulationseinrichtung zugeordnet, dann ist es zweckmäßig, wenn das zusätzliche Messmittel im extrakorporalen Blutkreislauf stromab der Dialyseeinheit und stromauf der Substitutionsmediumzufuhrstelle angeordnet ist. Dieses zumindest eine zusätzliche Messmittel dient als zusätzliche Sicherheitsmaßnahme und kann entweder zum Erkennen von Fehlfunktionen des/der zur Regelung dienenden Messmittel/s fungieren oder es wird in die Regelung einbezogen.

Weiters kann es von Vorteil sein, wenn die Blutreinigungsvorrichtung zumindest ein zusätzliches pH-Messmittel umfasst, welches im extrakorporalen Blutkreislauf stromab der Dialyseeinheit angeordnet ist, wobei ein dem pH-Wert entsprechendes Signal der Alarmeinrichtung zuführbar ist, welche bei Überschreiten oder Unterschreiten eines vorgebbaren pH-Werts oder pH-Wertebereichs ein Alarmsignal auslöst. Ist der Blutreinigungsvorrichtung eine Citrat-Antikoagulationseinrichtung zugeordnet, dann ist es zweckmäßig, wenn das zumindest eine zusätzliche pH-Messmittel im extrakorporalen Blutkreislauf stromab der Substitutionsmediumzufuhrstelle angeordnet ist. Durch dieses zusätzliche pH-Messmittel stromab der Dialyseeinheit bzw. stromab der Substitutionsmediumzufuhrstelle wird der pH-Wert des im extrakorporalen Blutkreislauf zirkulierenden Blutes nochmals kontrolliert, bevor das Blut wieder in den Körper zurückgeführt wird. Eine pH-Messung an diesen Positionen stellt somit eine zusätzliche Sicherheitsmaßnahme dar. Insbesondere kann bei vorhandener Citrat-Antikoagulation eine Falschdosierung des Substitutionsmediums, die zu Störungen des Säurebasenhaushalts und zu pH-Wert-Entgleisungen führen kann, rasch erkannt werden.

Die oben beschriebene erfindungsgemäße Blutreinigungsvorrichtung samt ihren Weiterbildungen kommt mit Vorteil zum Vorbeugen oder zum Behandeln von Störungen des Säurebasengleichgewichts während einer extrakorporalen Blutreinigung zur Anwendung. Störungen des Säurebasengleichgewichts umfassen eine Azidose und eine Alkalose. Die extrakorporale Blutreinigungsvorrichtung kann nach dem Prinzip der Hämodialyse, der Hämofiltration und der Hämodiafiltration, wie weiter oben beschrieben, und wahlweise in Kombination mit einer Adsorptionstechnologie, beruhen. Vorteilhafterweise wird die extrakorporale Blutreinigung in Kombination mit einer Antikoagulation mit Citrat durchgeführt, bei welcher die erfindungsgemäße Blutreinigungsvorrichtung besonders vorteilhafte Wirkungen in Bezug auf die Patientensicherheit und die Wiederherstellung bzw. Wahrung des Wohlbefindens zeigt.

Die Erfindung samt weiteren Vorzügen wird im Folgenden anhand von Zeichnungen mit nicht-einschränkenden Ausführungsbeispielen näher erläutert:
Die Zeichnungen zeigen:
   Fig. 1 eine schematische Darstellung einer erfindungsgemäßen Blutreinigungsvorrichtung ohne Citrat-Antikoagulation,
   Fig. 2 Varianten der in Fig. 1 gezeigten Blutreinigungsvorrichtung mit zusätzlichen Komponenten,
   Fig. 3 eine schematische Darstellung einer erfindungsgemäßen Blutreinigungsvorrichtung mit Citrat-Antikoagulation, und
   Fig. 4 Varianten der in Fig. 3 gezeigten Blutreinigungsvorrichtung mit zusätzlichen Komponenten.

Die Signalverbindungen der einzelnen Vorrichtungsbauteile miteinander bzw. mit den Steuerungen, Regel- und Alarmeinrichtungen sind in den Figuren durch gestrichelte Linien symbolisiert.

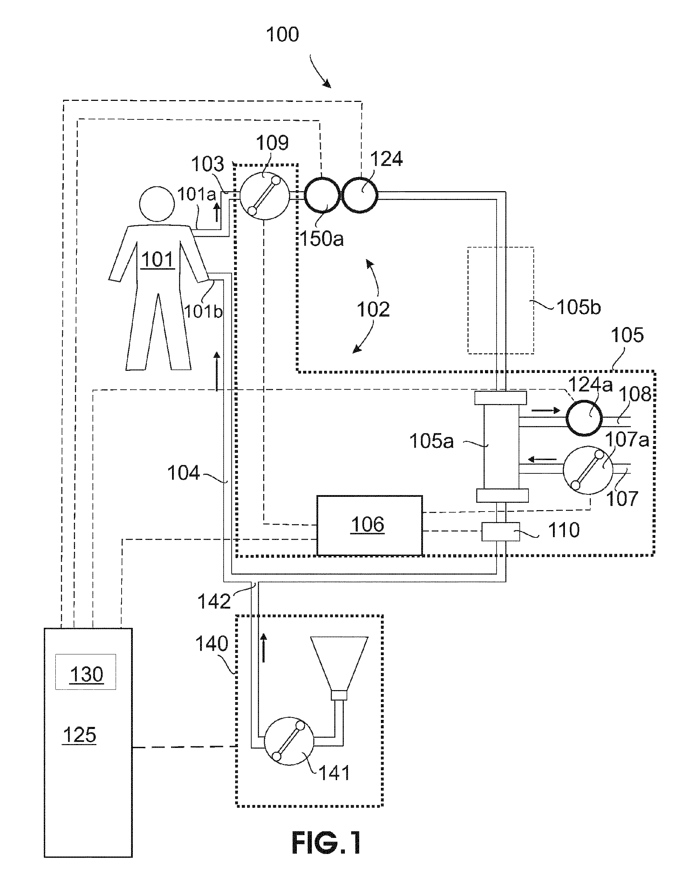

Fig. 1 zeigt eine schematische Darstellung einer erfindungsgemäßen Blutreinigungsvorrichtung 100 ohne Citrat-Antikoagulationseinrichtung. Die Antikoagulation erfolgt hier beispielsweise mit Heparin. Das Blut des Patienten 101 gelangt über einen arteriellen Zugang 101a in den extrakorporalen Blutkreislauf 102 mit einem Blutzulauf 103 (arterieller Schenkel), einem Blutablauf 104 (venöser Schenkel) und einer dazwischen liegenden Dialyseeinheit 105 (durch eine gestrichelte Umrandung dargestellt), und fließt über einen venösen Zugang 101b wieder in den Patienten 101 zurück. Die Dialyseeinheit 105 setzt sich im Wesentlichen aus einem Dialysefilter 105a (auch als Dialysator 105a bezeichnet), einer Blutpumpe 109, einer Luftfalle mit Luftblasendetektor 110 und einer Steuerung 106 zusammen. Je nach Blutreinigungssystem und erforderlicher Behandlung kann stromauf des Dialysefilters 105a noch ein zusätzliches Blutreinigungselement 105b - beispielsweise ein Hämofilter, ein Hämofilter mit geschlossenem Plasmakreislauf oder ein Adsorberkreislauf - vorgesehen sein. Im Dialysefilter 105a wird das Blut über eine semipermeable Membran mit der Dialysierlösung in Verbindung gebracht. Die Dialysierlösung wird üblicherweise vom Dialysegerät hergestellt, indem ein oder mehr Konzentrate mit Wasser verdünnt werden. In diesem Beispiel wird die Dialysierlösung aus einer konzentrierten sauren Komponente (z.B. Acetatlösung) und einer konzentrierten basischen Komponente (Lösung enthaltend Bicarbonat), weiteren für Dialysierlösungen typischen Komponenten und Wasser zusammengemischt (Mischvorrichtung nicht gezeigt). Durch Erhöhen oder Drosseln der Zufuhr an Bicarbonat-Lösung lässt sich die Konzentration des Bicarbonats in der Dialysierlösung entsprechend variieren. Mittels einer Dialysatpumpe 107a wird die Dialysierlösung über einen Dialysierlösungszulauf 107 in den Dialysefilter 105a gepumpt und nach Durchlauf des Dialysefilters 105a über einen Dialysatablauf 108 abgeleitet und entsorgt. Das Blut wird vom Patienten kommend mittels der Blutpumpe 109 durch den extrakorporalen Blutkreislauf 102 gepumpt, wobei über die Blutpumpe 109 die Höhe des Blutflusses Q_{B} eingestellt wird. Der Blutfluss Q_{B} [ml/min] stellt das Blutvolumen, das pro Zeiteinheit von der Pumpe gepumpt wird, dar. Typischerweise wird bei der Hämodialyse ein Blutfluss Q_{B} = 50 - 400 ml/min eingestellt. Bevor das Blut wieder in den Patienten 101 gelangt, passiert es noch eine Luftfalle mit Luftblasendetektor 110 zur Verhinderung einer Luftembolie. Die oben beschriebenen Bauteile bilden eine typische Ausführung eines extrakorporalen Blutreinigungssystems.

Der Blutreinigungsvorrichtung 100 ist erfindungsgemäß ein Regelkreis zur Regelung des Säurebasengleichgewichts des Blutes zugeordnet. Hierfür ist dem Regelkreis ein Messmittel zur Messung eines Blutpufferparameters (Bicarbonat-Sensor) zugeordnet.

Bei einer ersten in Fig. 1 gezeigten Ausführungsform ist im Blutzulauf 103 des extrakorporalen Blutkreislaufs 102 stromauf des Dialysefilters 105a ein Bicarbonat-Sensor 124 (HCO₃⁻ - Sensor 124) angeordnet, der die Bicarbonat-Konzentration des Bluts misst. Dabei ist es unwesentlich, ob der Bicarbonat-Sensor 124 stromab oder stromauf der Blutpumpe 109 angeordnet ist.

Alternativ zur ersten Ausführungsform kann der Bicarbonat-Sensor bei einer zweiten Ausführungsform im Dialysatablauf 108 angeordnet sein (Bicarbonat-Sensor 124a).

Die Messungen der Bicarbonat-Konzentration werden in regelmäßigen Zeitabständen durchgeführt, wobei der Zeitabstand so gewählt wird, dass ein Trend in Richtung eines unphysiologischen Zustands (Azidose, Alkalose) des Patienten frühzeitig erkannt wird. Bei den meisten Anwendungen ist ein Zeitintervall von 30 bis 60 Minuten ausreichend. Bei gewissen Patientengruppen ist der Zeitabstand zwischen zwei aufeinanderfolgenden Messungen kürzer gewählt z.B. 15 Minuten.

Für die Regelung und die Konstanthaltung des Säurebasengleichgewichts des Blutes wird ein einfacher Regelmechanismus geschaffen. Der Bicarbonat-Sensor 124 bzw. 124a ist mit einer Regeleinrichtung 125 mittels Signalverbindung verbunden. Der vom Bicarbonat-Sensor 124 bzw. 124a gemessene Istwert wird als ein der Bicarbonat-Konzentration des Blutes entsprechendes Signal der Regeleinrichtung 125 zugeführt, welche den Istwert des Blutpufferparameters über die Zufuhr von Bicarbonat auf einen vorgebbaren Sollwert oder Sollbereich regelt. Wie oben beschrieben, wird Bicarbonat zugeführt, um die üblicherweise bei Dialysepatienten vorherrschende Azidose im Zuge der Dialyse zu beseitigen bzw. um das Säurebasengleichgewicht während der Dialyse in gewünschten, sehr engen physiologischen Bereich konstant zu halten. In der Fig. 1 sind zwei Möglichkeiten der Regelung der Bicarbonat-Zufuhr aufgezeigt:
*) Bei der ersten Möglichkeit der Regelung wird das Bicarbonat mittels der Dialysierlösung dem Blut zugeführt und stellt eine bevorzugte Ausführungsform bei der Hämodialyse und der Hämodiafiltration dar. Entsprechend des gemessenen Istwerts durch den Bicarbonat-Sensor 124 bzw. 124a wird daher die Bicarbonat-Konzentration in der Dialysierflüssigkeit entsprechend erhöht oder verringert. Die Regeleinrichtung 125 ist hierfür mit der Steuerung 106 der Dialyseeinheit 105 über eine Signalverbindung verbunden. Unterschreitet die gemessene Bicarbonat-Konzentration im Blut den vorgebbaren Sollbereich, der z.B. als 21-26 mmol/l vorgegeben ist, dann liegt eine Übersäuerung (Azidose) vor und die Konzentration des Bicarbonats in der Dialysierlösung wird erhöht, indem beim Zusammenmischen der Dialysierlösung mehr konzentrierte basische Komponente zugemischt wird. Im Fall einer Alkalose, d.h. wenn die gemessene Bicarbonat-Konzentration im Blut den Sollbereich überschreitet, wird entsprechend weniger oder gar keine konzentrierte basische Komponente zugemischt, wodurch sich die Bicarbonat-Konzentration in der Dialysierlösung erniedrigt.
*) Eine zweite Möglichkeit der Regelung der Bicarbonatzufuhr, die insbesondere bei der Hämofiltration und der Hämodiafiltration Anwendung findet, ist eine separate Infusion einer wässrigen Bicarbonat-Substitutionslösung mittels einer Bicarbonat-Dosiereinrichtung 140, wobei die Regeleinrichtung 125 die Bicarbonat-Pumpe 141 ansteuert und die Dosierung des Bicarbonats entsprechend dem durch den Bicarbonat-Sensor 124 bzw. 124a gemessenen Istwert variiert. Wie in der Fig. 1 gezeigt wird die wässrige Bicarbonat-Lösung stromab des Dialysefilters 105a in den Blutablauf 104 an einer Bicarbonat-Zufuhrstelle 142 infundiert. Beispielsweise ist es bei der "Online-Hämofiltration" vorgesehen, dass aus dem Dialysat eine Bicarbonat-enthaltende Substitutionslösung zubereitet wird, die steril dem Patienten durch Infusion in den extrakorporalen Blutkreislauf zugeführt wird.

Es ist grundsätzlich auch möglich, die wässrige Bicarbonat-Lösung dem Patienten 101 über einen venösen Zugang (nicht gezeigt) zuzuführen, allerdings ist diese Option weniger bevorzugt, da ein zusätzlicher venöser Zugang beim Patienten vorgesehen ist.

Es ist weiters möglich, die zuvor beschriebenen zwei Möglichkeiten miteinander zu kombinieren, beispielsweise durch Einstellen einer geringeren Bicarbonatkonzentration im Dialysat und einer geregelten Zugabe über die Zufuhr der Substitutionslösung.

Stromauf des Dialysefilters 105a ist erfindungsgemäß ferner ein pH-Sensor 150a angeordnet. Der an dieser Position gemessene pH-Wert entspricht somit dem intrakorporal vorliegenden pH-Wert des Blutes. Der pH-Sensor stellt eine wichtige Sicherheitseinrichtung dar und ermöglicht das Unterscheiden zwischen einer kompensierten und einer nicht-kompensierten Alkalose bzw. Azidose und ein rasches Erkennen lebensbedrohlicher Störungen des Säurebasengleichgewichts. Der pH-Sensor 150a ist mit einer der Regeleinrichtung 125 zugeordneten Alarmeinrichtung 130 über Signalverbindungen verbunden. Bei Überschreiten oder Unterschreiten eines vorgebbaren pH-Werts oder pH-Wertebereichs wird durch die Alarmeinrichtung 130 ein Alarmsignal ausgelöst, welches einen Bediener, z.B. einen Arzt, auf den Zustand einer nicht-kompensierten Alkalose bzw. einer nicht-kompensierten Azidose aufmerksam macht. Das Alarmsignal kann beispielsweise ein akustisches oder optisches Signal sein. Der Alarm kann weiters ein Anhalten der gesamten Blutreinigungsvorrichtung durch Abschalten der Pumpen 109 und 141 bewirken.

In den Fig. 1 bis 4 ist jeweils nur ein Bicarbonat-Sensor bzw. pH-Sensor an einer bestimmten Position in der Blutreinigungsvorrichtung angeordnet. Es können sich jedoch an jeder Position auch mehrere wesensgleiche Sensoren, die alternierend oder zeitgleich arbeiten, befinden.

Anstelle der in den Figuren 1 bis 4 gezeigten Bicarbonat-Sensoren können auch Sensoren zur Messung des CO₂-Partialdrucks (pCO₂-Sensoren) verwendet werden. Bicarbonat-Sensoren bzw. Sensoren zur Messung des CO₂-Partialdrucks, wie sie in der Erfindung und den gezeigten Beispielen der Fig. 1 bis 4 gezeigt sind, sind beispielsweise in der EP 0759 551 B1, CA 2173672 A2 und der US 2009/027095 A1 geoffenbart.

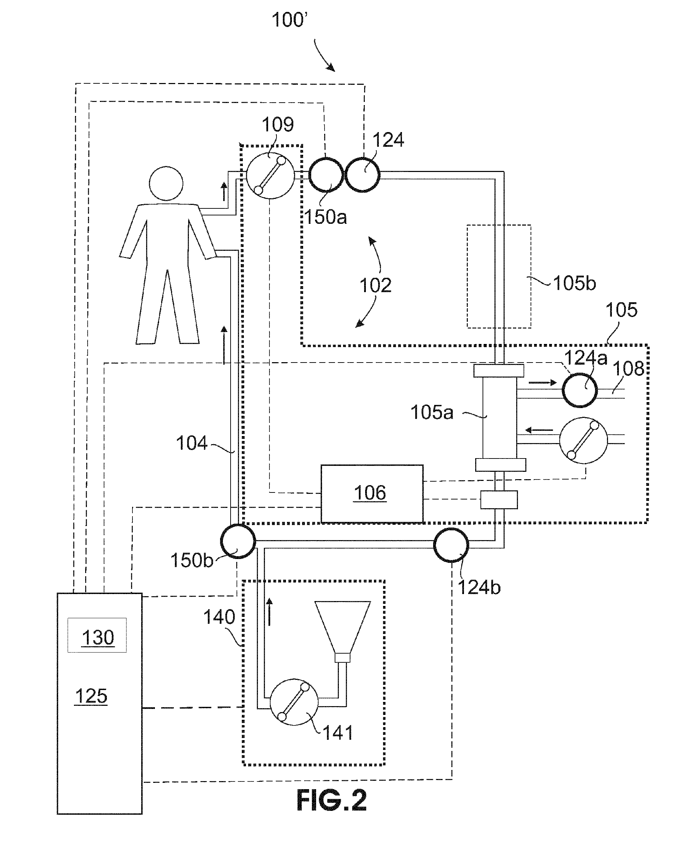

Fig. 2 zeigt Varianten der in Fig. 1 gezeigten Blutreinigungsvorrichtung 100 mit zusätzlichen Komponenten. Die in Fig. 2 gezeigte Vorrichtung 100'entspricht jener der Fig. 1, sofern im Folgenden nicht anders angegeben. Die dargestellten Erweiterungen können einzeln oder in Kombination zur Anwendung kommen.

Als Weiterbildung der oben genannten Ausführungsformen, in welchen zumindest ein erster Bicarbonat-Sensor in einer ersten Position angeordnet ist, kann als weiterführende Sicherheitsmaßnahme dem Regelkreis an einer oder mehreren weiteren Positionen zumindest ein zusätzlicher Bicarbonat-Sensor zugeordnet sein. Der zusätzliche Bicarbonat-Sensor an einer oder mehreren weiteren Positionen dient entweder zum Erkennen von Fehlfunktionen des zur Regelung fungierenden ersten Bicarbonat-Sensors oder kann wie der Bicarbonat-Sensor an der ersten Position ebenfalls in die Regelung wie oben beschrieben einbezogen sein.

Bei einer ersten in Fig. 2 gezeigten Variante ist bei Vorhandensein eines Bicarbonat-Sensors 124 stromauf des Dialysefilters dem Regelkreis ein im Dialysatablauf 108 angeordneter Bicarbonat-Sensor 124a zugeordnet. Alternativ ist bei einer zweiten Variante bei Vorhandensein eines Bicarbonat-Sensors 124a im Dialysatablauf 108 dem Regelkreis im extrakorporalen Blutkreislauf 102 stromauf der Dialyseeinheit 105a ein Bicarbonat-Sensor 124 zugeordnet.

In einem weiteren Aspekt kann ein zusätzlicher Bicarbonat-Sensor 124b stromab der Dialyseeinheit 105a angeordnet sein. An dieser Position im extrakorporalen Blutkreislauf 102 sollte wieder ein im physiologischen Bereich liegender Säurebasenhaushalt vorliegen.

In einem weiteren Aspekt ist zusätzlich zum pH-Sensor 150a ein weiterer pH-Sensor 150b im Blutablauf 104 stromab des Dialysefilters 105a angeordnet, wenn Bicarbonat über die Dialyseflüssigkeit zugeführt wird. Wird das Bicarbonat über eine Substitutionslösung zugeführt, so ist der pH-Sensor zweckmäßigerweise im Blutablauf 104 stromab der Bicarbonat-Zufuhrstelle 142 angeordnet. Dies ermöglicht eine Kontrolle des pH-Werts des Blutes im extrakorporalen Blutkreislauf kurz bevor das Blut wieder in den Patienten gelangt. Beispielsweise können Falschdosierungen des Bicarbonats in der Dialysierflüssigkeit oder in der Substitutionsflüssigkeit rasch erkannt werden.

Wie der pH-Sensor 150a ist auch der pH-Sensor 150b mit einer der Regeleinrichtung 125 zugeordneten Alarmeinrichtung 130 über Signalverbindungen verbunden. Bei Überschreiten oder Unterschreiten eines vorgebbaren pH-Werts oder pH-Wertebereichs wird durch die Alarmeinrichtung 130 ein Alarmsignal ausgelöst, welches einen Bediener, z.B. einen Arzt, auf den Zustand einer nicht-kompensierten Alkalose bzw. einer nicht-kompensierten Azidose aufmerksam macht. Das Alarmsignal kann beispielsweise ein akustisches oder optisches Signal sein. Der Alarm kann weiters ein Anhalten der gesamten Blutreinigungsvorrichtung durch Abschalten der Pumpen 109 und 141 bewirken.

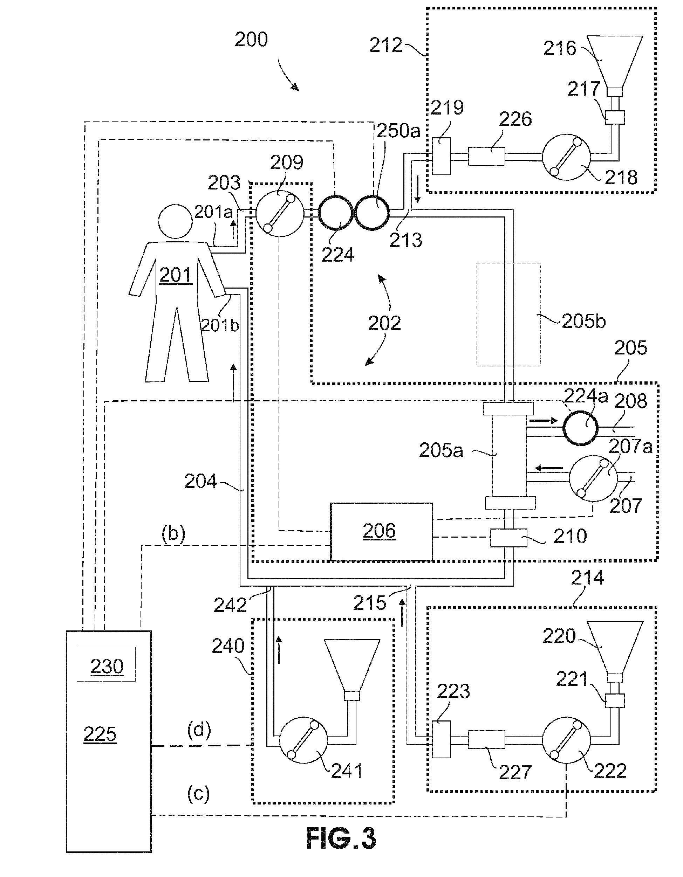

Fig. 3 zeigt eine schematische Darstellung einer erfindungsgemäßen Blutreinigungsvorrichtung 200 mit einer automatisierten Citrat-Antikoagulation. Das Blut des Patienten 201 gelangt über einen arteriellen Zugang 201a in den extrakorporalen Blutkreislauf 202 mit einem Blutzulauf 203 (arterieller Schenkel), einem Blutablauf 204 (venöser Schenkel) und einer dazwischen liegenden Dialyseeinheit 205 (durch eine gestrichelte Umrandung dargestellt), und fließt über einen venösen Zugang 201b wieder in den Patienten zurück. Die Dialyseeinheit 205 setzt sich im Wesentlichen aus einem Dialysefilter 205a, einer Blutpumpe 209, einer Luftfalle mit Luftblasendetektor 210 und einer Steuerung 206 zusammen. Je nach Blutreinigungssystem und erforderlicher Behandlung kann stromauf des Dialysefilters 205a noch ein zusätzliches Blutreinigungselement 205b - beispielsweise ein Hämofilter, ein Hämofilter mit geschlossenem Plasmakreislauf oder ein Adsorberkreislauf - vorgesehen sein. Im Dialysefilter 205a wird das Blut über eine semipermeable Membran mit der Dialysierlösung in Verbindung gebracht. Die Dialysierlösung wird üblicherweise vom Dialysegerät hergestellt, indem ein oder mehr Konzentrate mit Wasser verdünnt werden. In diesem Beispiel kann die Dialysierlösung aus einer konzentrierten sauren Komponente (z.B. Acetatlösung) und einer konzentrierten basischen Komponente (Lösung enthaltend Bicarbonat), weiteren für Dialysierlösungen typischen Komponenten und Wasser zusammengemischt (Mischvorrichtung nicht gezeigt). Durch Erhöhen oder Drosseln der Zufuhr an Bicarbonat-Lösung lässt sich die Konzentration des Bicarbonats in der Dialysierlösung entsprechend variieren. Mittels einer Dialysatpumpe 207a wird die Dialysierlösung über einen Dialysierlösungszulauf 207 in den Dialysefilter 205a gepumpt und nach Durchlauf des Dialysefilters 205a über einen Dialysatablauf 208 abgeleitet und entsorgt. Das Blut wird vom Patienten kommend mittels der Blutpumpe 209 durch den extrakorporalen Blutkreislauf 202 gepumpt, wobei über die Blutpumpe 209 die Höhe des Blutflusses Q_{B} eingestellt wird. Der Blutfluss Q_{B} [ml/min] stellt das Blutvolumen, das pro Zeiteinheit von der Pumpe gepumpt wird, dar. Typischerweise wird bei der Hämodialyse ein Blutfluss Q_{B} = 50 - 400 ml/min eingestellt. Bevor das Blut wieder in den Patienten 201 gelangt, passiert es noch eine Luftfalle mit Luftblasendetektor 210 zur Verhinderung einer Luftembolie. Die oben beschriebenen Bauteile bilden eine typische Ausführung eines extrakorporalen Blutreinigungssystems.

Die Blutreinigungsvorrichtung 200 weist ferner eine Vorrichtung zur Citrat-Antikoagulation mit einer gesteuerten Citrat-Dosiereinrichtung 212 zur Zuführung von Citrat an einer Citratzufuhrstelle 213 stromauf des Dialysefilters 205a (und des Blutreinigungselements 205b, wenn vorhanden) und einer gesteuerten Substitutionsmedium-Dosiereinrichtung 214 zur Zuführung eines Substitutionsmediums an einer Substitutionsmedium-Zufuhrstelle 215 stromab des Dialysefilters 205a. Die Citrat-Dosiereinrichtung 212 weist einen Behälter mit Citratlösung 216, einen Tropfendetektor 217, eine Citratpumpe 218, einen Drucksensor 226 und einen Luftblasendetektor 219 auf. Durch die Infusion der Citratlösung, welche entweder in Form von Na₃-Citrat oder ACD-A (acid citrate dextrose-A), das neben Citrat auch Zitronensäure und Dextrose enthält, an der Citratzufuhrstelle 213 in den extrakorporalen Blutkreislauf 202, wird das Blut in seiner Gerinnung gehemmt. Ähnlich der Citrat-Dosiereinrichtung 212, beinhaltet die Substitutionsmedium-Dosiereinrichtung 214 ebenfalls ein Behältnis mit Substitutionsmedium 220, einen Tropfendetektor 221, eine Substitutionsmediumspumpe 222, einen Drucksensor 227 und einen Luftblasendetektor 223. Das Substitutionsmedium enthält vorwiegend Ca-Ionen oder Ca- und Mg-Ionen und wird an der Subsitutionsmedium-Zufuhrstelle 215 in den extrakorporalen Blutkreislauf 202 infundiert. Die Pumpendrehzahl der Pumpen 218, 222 wird mit in den Pumpen 218, 222 integrierten Hallsensoren und Inkrementalaufnehmern ermittelt und überwacht.

Der Blutreinigungsvorrichtung 200 ist erfindungsgemäß ein Regelkreis zur Regelung des Säurebasengleichgewichts des Blutes zugeordnet. Hierfür ist dem Regelkreis ein Messmittel zur Messung eines Blutpufferparameters (Bicarbonat-Sensor) zugeordnet.

Bei einer ersten in Fig. 3 gezeigten Ausführungsform ist im Blutzulauf 203 des extrakorporalen Blutkreislaufs 202 stromauf der Citratzufuhrstelle 213 ein Bicarbonat-Sensor 224 (HCO₃⁻ - Sensor 224) angeordnet, der die Bicarbonat-Konzentration des Bluts misst. Dabei ist es unwesentlich, ob der Bicarbonat-Sensor 224 stromab oder stromauf der Blutpumpe 209 angeordnet ist.

Alternativ zur ersten Ausführungsform kann der Bicarbonat-Sensor bei einer zweiten Ausführungsform im Dialysatablauf 208 angeordnet sein (Bicarbonat-Sensor 224a).

Die Messungen der Bicarbonat-Konzentration werden in regelmäßigen Zeitabständen durchgeführt, wobei der Zeitabstand so gewählt wird, dass ein Trend in Richtung eines unphysiologischen Zustands (Azidose, Alkalose) des Patienten frühzeitig erkannt wird. Bei den meisten Anwendungen ist ein Zeitintervall von 30 bis 60 Minuten ausreichend. Bei gewissen Patientengruppen, z.B. Patienten mit Lebererkrankungen, welche das zugeführte Citrat nur beschränkt in Bicarbonat umwandeln können, ist der Zeitabstand zwischen zwei aufeinanderfolgenden Messungen kürzer gewählt z.B. 15 Minuten.

Für die Regelung und die Konstanthaltung des Säurebasengleichgewichts des Blutes wird ein einfacher Regelmechanismus geschaffen. Der Bicarbonat-Sensor 224 bzw. 224a ist mit einer Regeleinrichtung 225 mittels Signalverbindung verbunden. Der vom Bicarbonat-Sensor 224 bzw. 224a gemessene Istwert wird als ein der Bicarbonat-Konzentration des Blutes entsprechendes Signal der Regeleinrichtung 225 zugeführt, welche den Istwert des Blutpufferparameters über die Zufuhr von Bicarbonat auf einen vorgebbaren Sollwert oder Sollbereich regelt. Wie oben beschrieben, wird Bicarbonat zugeführt, um die üblicherweise bei Dialysepatienten vorherrschende Azidose im Zuge der Dialyse zu beseitigen bzw. um das Säurebasengleichgewicht während der Dialyse in gewünschten, sehr engen physiologischen Bereich konstant zu halten. In der Fig. 3 sind drei Möglichkeiten der Regelung der Bicarbonat-Zufuhr aufgezeigt:
*) Bei der ersten Möglichkeit der Regelung wird das Bicarbonat mittels der Dialysierlösung dem Blut zugeführt. Die erste Möglichkeit der Regelung stellt eine bevorzugte Ausführungsform bei der Hämodialyse und der Hämodiafiltration dar. Entsprechend des gemessenen Istwerts durch den Bicarbonat-Sensor 224 oder 224a wird daher die Bicarbonat-Konzentration in der Dialysierflüssigkeit entsprechend erhöht oder verringert. Die Regeleinrichtung 225 ist hierfür mit der Steuerung 206 der Dialyseeinheit 205 verbunden. Unterschreitet die gemessene Bicarbonat-Konzentration im Blut den vorgebbaren Sollbereich, der z.B. als 21-26 mmol/l vorgegeben ist, dann liegt eine Übersäuerung (Azidose) vor und die Konzentration des Bicarbonats in der Dialysierlösung wird erhöht, indem beim Zusammenmischen der Dialysierlösung mehr konzentrierte basische Komponente zugemischt wird. Im Fall einer Alkalose, d.h. wenn die gemessene Bicarbonat-Konzentration im Blut den Sollbereich überschreitet, wird entsprechend weniger oder gar keine konzentrierte basische Komponente zugemischt, wodurch sich die Bicarbonat-Konzentration in der Dialysierlösung erniedrigt.
*) Bei der zweiten Möglichkeit der Regelung wird das Bicarbonat mittels des Substitutionsmediums der Citrat-Antikoagulationseinrichtung zugeführt, welches neben den anderen Ionen wie Calciumionen oder Magnesiumionen auch Bicarbonat enthält. Die Regeleinrichtung 225 regelt in Abhängigkeit der von Bicarbonat-Sensor 224 bzw. 224a gemessenen Bicarbonat-Konzentration die Bicarbonat-Zufuhr und damit das Säurebasengleichgewicht durch Ansteuern der Substitutionsmediumspumpe 222 der Substitutionsmedium-Dosiereinrichtung 214. Diese Möglichkeit wird weniger bevorzugt, da durch Änderung der Zufuhr des Substitutionsmediums nicht nur die Bicarbonat-Zufuhr, sondern auch die Zufuhr der anderen wichtigen Ionen (Calcium- und Magnesiumionen) geändert wird, was in bestimmten Fällen zu physiologischen Ungleichgewichten im Elektrolythaushalt (z.B. Hypocalcämie und Hypercalcämie) führen kann.
*) Eine dritte Möglichkeit der Regelung der Bicarbonatzufuhr, die insbesondere bei der Hämofiltration und der Hämodiafiltration Anwendung findet, ist eine separate Infusion einer wässrigen Bicarbonat-Substitutionslösung mittels einer Bicarbonat-Dosiereinrichtung 240, wobei die Steuerung 225 die Bicarbonat-Pumpe 241 ansteuert und die Dosierung des Bicarbonats entsprechend dem durch den Bicarbonat-Sensor 224 gemessenen Istwert variiert. Dadurch können die Nachteile der zweiten Möglichkeit (Ausfällen von Calcium- und Magnesiumcarbonat) umgangen werden. Wie in der Fig. 3 gezeigt wird die wässrige Bicarbonat-Lösung in den Blutablauf 204 an einer Bicarbonat-Zufuhrstelle 242 infundiert. Beispielsweise ist es bei der "Online-Hämofiltration" vorgesehen, dass aus dem Dialysat eine Substitutionslösung zubereitet wird, die steril dem Patienten durch Infusion in den extrakorporalen Blutkreislauf zugeführt wird.

Es ist grundsätzlich auch möglich, die wässrige Bicarbonat-Lösung dem Patienten 201 über einen venösen Zugang (nicht gezeigt) zuzuführen, allerdings ist diese Option weniger bevorzugt.

Es ist weiters möglich, die zuvor beschriebenen drei Möglichkeiten miteinander zu kombinieren, zB. durch Einstellen einer geringeren Bicarbonatkonzentration im Dialysat und einer geregelten Zugabe über das Substitutionsmedium.

Stromauf der Citratzufuhrstelle 213 ist im Blutzulauf 203 erfindungsgemäß ferner ein pH-Sensor 250a angeordnet. Der an dieser Position gemessene pH-Wert entspricht somit dem intrakorporal vorliegenden pH-Wert des Blutes. Der pH-Sensor stellt eine wichtige Sicherheitseinrichtung dar und ermöglicht das Unterscheiden zwischen einer kompensierten und einer nicht kompensierten Alkalose bzw. Azidose und ein rasches Erkennen lebensbedrohlicher Störungen des Säurebasengleichgewichts. Der pH-Sensor 250a ist mit einer der Regeleinrichtung 225 zugeordneten Alarmeinrichtung 230 über Signalverbindungen verbunden. Bei Überschreiten oder Unterschreiten eines vorgebbaren pH-Werts oder pH-Wertebereichs wird durch die Alarmeinrichtung 230 ein Alarmsignal ausgelöst, welches einen Bediener, z.B. einen Arzt, auf den Zustand einer nicht-kompensierten Alkalose bzw. einer nicht-kompensierten Azidose aufmerksam macht. Das Alarmsignal kann beispielsweise ein akustisches oder optisches Signal sein. Der Alarm kann weiters ein Anhalten der gesamten Blutreinigungsvorrichtung durch Abschalten der Pumpen 209, 218, 222 und 241 bewirken.

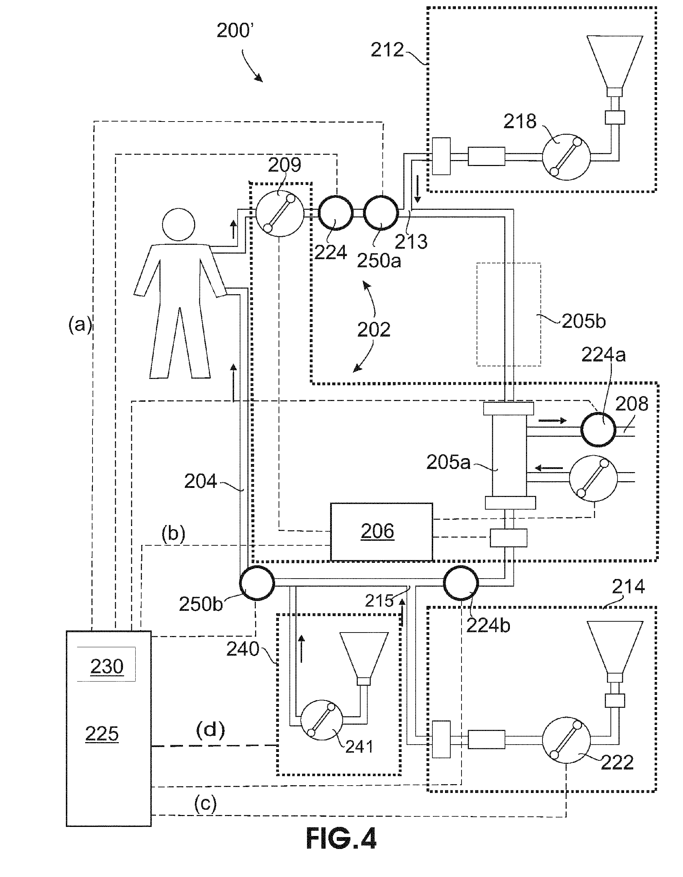

Fig. 4 zeigt Varianten der in Fig. 3 gezeigten Blutreinigungsvorrichtung 200 mit zusätzlichen Komponenten. Die in Fig. 4 gezeigte Vorrichtung 200'entspricht jener der Fig. 3, sofern im Folgenden nicht anders angegeben. Die dargestellten Erweiterungen können einzeln oder in Kombination zur Anwendung kommen.

Als Weiterbildung der in Fig. 3 gezeigten Ausführungsformen und Varianten, in welchen zumindest ein erster Bicarbonat-Sensor in einer ersten Position angeordnet ist, kann als weiterführende Sicherheitsmaßnahme dem Regelkreis an einer oder mehreren weiteren Positionen zumindest ein zusätzlicher Bicarbonat-Sensor zugeordnet sein. Der zusätzliche Bicarbonat-Sensor an einer oder mehreren weiteren Positionen dient entweder zum Erkennen von Fehlfunktionen des zur Regelung fungierenden ersten Bicarbonat-Sensors oder kann wie der Bicarbonat-Sensor an der ersten Position ebenfalls in die Regelung wie oben beschrieben einbezogen sein.

Bei einer ersten in Fig. 4 gezeigten Variante ist bei Vorhandensein eines Bicarbonat-Sensors 224 stromauf der Citratzufuhrstelle 213 dem Regelkreis ein im Dialysatablauf 208 angeordneter Bicarbonat-Sensor 224a zugeordnet. Alternativ ist bei einer zweiten Variante bei Vorhandensein eines Bicarbonat-Sensors 224a im Dialysatablauf 208 dem Regelkreis im extrakorporalen Blutkreislauf 202 stromauf der Citratzufuhrstelle 213 ein Bicarbonat-Sensor 224 zugeordnet.

In einem weiteren Aspekt kann ein zusätzlicher Bicarbonat-Sensor 224b stromab der Dialyseeinheit 205a und stromauf der Substitutionsmediumszufuhrstelle 215 angeordnet sein. An dieser Position im extrakorporalen Blutkreislauf 202 sollte wieder ein im physiologischen Bereich liegender Säurebasenhaushalt vorliegen.

In einem weiteren Aspekt ist zusätzlich zum pH-Sensor 250a ein weiterer pH-Sensor 250b im Blutablauf 204 angeordnet, wenn Bicarbonat über die Dialyseflüssigkeit zugeführt wird. Wird das Bicarbonat über eine Substitutionslösung zugeführt, so ist der pH-Sensor zweckmäßigerweise nach Zugabe des Bicarbonats im Blutablauf 204 stromab der Substitutionsmediumszufuhrstelle 215 bzw. der Bicarbonat-Zufuhrstelle 142 angeordnet. Dies ermöglicht eine Kontrolle des pH-Werts des Blutes im extrakorporalen Blutkreislauf kurz bevor das Blut wieder in den Patienten gelangt. Beispielsweise können Falschdosierungen des Bicarbonats und/oder des Citrats in der Dialysierflüssigkeit, der Citratlösung und in den Substitutionsflüssigkeiten rasch erkannt werden.

Wie der pH-Sensor 250a ist auch der pH-Sensor 250b mit einer der Regeleinrichtung 125 zugeordneten Alarmeinrichtung 230 über Signalverbindungen verbunden. Bei Überschreiten oder Unterschreiten eines vorgebbaren pH-Werts oder pH-Wertebereichs wird durch die Alarmeinrichtung 230 ein Alarmsignal ausgelöst, welches einen Bediener, z.B. einen Arzt, auf den Zustand einer nicht-kompensierten Alkalose bzw. einer nicht-kompensierten Azidose aufmerksam macht. Das Alarmsignal kann beispielsweise ein akustisches oder optisches Signal sein. Der Alarm kann weiters ein Anhalten der gesamten Blutreinigungsvorrichtung durch Abschalten der Pumpen 209, 218, 222 und 241 bewirken.

Die oben beschriebenen Verwirklichungen sind nur Beispiele unter Vielen und sind folglich nicht als einschränkend zu betrachten.

## Patentansprüche

1. Blutreinigungsvorrichtung (100,100',200,200') aufweisend:
einen extrakorporalen Blutkreislauf (102,202) mit einem Blutzulauf (103,203) zu einer Dialyseeinheit (105,205) für von einem Patienten entnommenes Blut und einem Blutablauf (104,204) von der Dialyseeinheit (105,205) für in den Patienten zurückzuführendes Blut, wobei die Dialyseeinheit (105,205) einen Zulauf (107,207) für eine Dialysierlösung und einen Dialysatablauf (108,208) umfasst,
**gekennzeichnet durch**
einen Regelkreis zur Regelung des Säurebasengleichgewichts des Blutes, wobei dem Blut mittels einer wässrigen Bicarbonat-hältigen Lösung Bicarbonat zugeführt ist, wobei dem Regelkreis zumindest ein Messmittel (124,124a,224,224a) zum Messen eines Blutpufferparameters zugeordnet ist, wobei der Blutpufferparameter ausgewählt ist aus der Gruppe bestehend aus Bicarbonat-Konzentration und CO₂-Partialdruck, und wobei ein dem gemessenen Istwert des Blutpufferparameters entsprechendes Signal einer Regeleinrichtung (125,225) zuführbar ist, welche den Istwert des Blutpufferparameters über die Zufuhr des Bicarbonats auf einen vorgebbaren Sollwert oder Sollwertbereich regelt, und
zumindest ein pH-Messmittel (150a,250a), welches im extrakorporalen Blutkreislauf (102,202) stromauf der Dialyseeinheit (105,205) angeordnet ist, wobei ein dem pH-Wert entsprechendes Signal einer Alarmeinrichtung (130,230) zuführbar ist, welche bei Überschreiten oder Unterschreiten eines vorgebbaren pH-Werts oder pH-Wert-Bereichs ein Alarmsignal auslöst.

2. Blutreinigungsvorrichtung nach Anspruch 1, **dadurch gekennzeichnet, dass** es sich bei der wässrigen Bicarbonat-hältigen Lösung um eine Dialysierlösung handelt.

3. Blutreinigungsvorrichtung nach Anspruch 1, **dadurch gekennzeichnet, dass** es sich bei der wässrigen Bicarbonat-hältigen Lösung um eine Infusionslösung handelt.

4. Blutreinigungsvorrichtung nach einem der Ansprüche 1 bis 3, **gekennzeichnet durch** eine Citrat-Antikoagulationseinsrichtung, wobei dem extrakorporalen Blutkreislauf (202) an einer Citratzufuhrstelle (213) stromauf der Dialyseeinheit (205) Citrat und an einer Substitutionsmediumzufuhrstelle (215) stromab der Dialyseeinheit (205) Substitutionsmedium zugeführt ist.

5. Blutreinigungsvorrichtung nach Anspruch 4, **dadurch gekennzeichnet, dass** das zumindest eine pH-Messmittel (250a) im extrakorporalen Blutkreislauf (202) stromauf der Citratzufuhrstelle (213) angeordnet ist.

6. Blutreinigungsvorrichtung nach einem der Ansprüche 1 bis 5, **dadurch gekennzeichnet, dass** das zumindest eine Messmittel (124,224) zum Messen eines Blutpufferparameters im extrakorporalen Blutkreislauf (102,202) stromauf der Dialyseeinheit (105,205) angeordnet ist.

7. Blutreinigungsvorrichtung nach Anspruch 6, **dadurch gekennzeichnet, dass** das zumindest eine Messmittel (224) zum Messen eines Blutpufferparameters im extrakorporalen Blutkreislauf (202) stromauf der Citratzufuhrstelle (213) angeordnet ist.

8. Blutreinigungsvorrichtung nach einem der Ansprüche 1 bis 5, **dadurch gekennzeichnet, dass** das zumindest eine Messmittel (124a,224a) zum Messen eines Blutpufferparameters im Dialysatablauf (108,208) angeordnet ist.

9. Blutreinigungsvorrichtung nach Anspruch 6 oder 7, **dadurch gekennzeichnet, dass** dem Regelkreis zumindest ein zusätzliches Messmittel (124a,224a) zum Messen eines Blutpufferparameters zugeordnet ist, welches im Dialysatablauf (108,208) angeordnet ist.

10. Blutreinigungsvorrichtung nach Anspruch 8, **dadurch gekennzeichnet, dass** dem Regelkreis zumindest ein zusätzliches Messmittel (124,224) zum Messen eines Blutpufferparameters zugeordnet ist, welches im extrakorporalen Blutkreislauf (102,202) stromauf der Dialyseeinheit (105,205) angeordnet ist.

11. Blutreinigungsvorrichtung nach Anspruch 10, **dadurch gekennzeichnet, dass** das zumindest eine zusätzliche Messmittel (224) zum Messen eines Blutpufferparameters im extrakorporalen Blutkreislauf (202) stromauf der Citratzufuhrstelle (213) angeordnet ist.

12. Blutreinigungsvorrichtung nach einem der Ansprüche 6 bis 11, **dadurch gekennzeichnet, dass** dem Regelkreis zumindest ein zusätzliches Messmittel (124b, 224b) zum Messen eines Blutpufferparameters zugeordnet ist, welches im extrakorporalen Blutkreislauf (102,202) stromab der Dialyseeinheit (105,205) angeordnet ist.

13. Blutreinigungsvorrichtung nach Anspruch 12, **dadurch gekennzeichnet, dass** das zumindest eine zusätzliche Messmittel (224b) zum Messen eines Blutpufferparameters im extrakorporalen Blutkreislauf (202) stromab der Dialyseeinheit (205) und stromauf der Substitutionsmediumzufuhrstelle (215) angeordnet ist.

14. Blutreinigungsvorrichtung nach einem der Ansprüche 1 bis 13, **gekennzeichnet durch** zumindest ein zusätzliches pH-Messmittel (150b, 250b), welches im extrakorporalen Blutkreislauf (102,202) stromab der Dialyseeinheit (105,205) angeordnet ist, wobei ein dem pH-Wert entsprechendes Signal der Alarmeinrichtung (130,203) zuführbar ist, welche bei Überschreiten oder Unterschreiten eines vorgebbaren pH-Werts oder pH-Wertebereichs ein Alarmsignal auslöst.

15. Blutreinigungsvorrichtung nach Anspruch 15, **dadurch gekennzeichnet, dass** das zumindest eine zusätzliche pH-Messmittel (250b) im extrakorporalen Blutkreislauf (202) stromab der Substitutionsmediumzufuhrstelle (215) angeordnet ist.

16. Blutreinigungsvorrichtung nach einem der Ansprüche 1 bis 15 zum Vorbeugen oder zum Behandeln von Störungen des Säurebasengleichgewichts, umfassend eine Azidose oder eine Alkalose, während einer extrakorporalen Blutreinigung.
